# EUROPEAN PATENT APPLICATION

(11) **EP 4 529 871 A2**
(43) Date of publication of application: **02.04.2025**
(21) Application number: 24200846.4
(22) Date of filing: 17.09.2024
(51) Int. Cl.: A61B 18/00

(54) **PORTABLE ABLATION APPARATUS**

(30) Priority: 28.09.2023 US 202318477388
(71) Applicant: Varian Medical Systems, Inc., Palo Alto, CA 94304 (US)
(72) Inventor: JESTER, Steve, Eden Prairie, 55346 (US); OLSON, Dane, New Hope, 55427 (US); HATTAN, Paul, Minneapolis, 55401 (US); BACHMAN, Tim, St. Paul, 55116 (US); GOULET, Matt, St. Paul, 55104 (US); WEBER, Charlie, St Paul, 55104 (US); ERNSTER, Logan, St. Paul, 55012 (US)
(74) Representative: Mathisen & Macara LLP

(57) **Abstract**

A microwave ablation apparatus includes a movable cart with a support surface and a portable base unit including at least one microwave generator and at least one coolant flow generator. The portable base unit is removably positioned on the support surface. The at least one ablation probe assembly is configured to couple to the at least one microwave generator and the at least one coolant flow generator.

## Description

### FIELD

The present disclosure relates to portable ablation apparatuses and related methods of use.

### BACKGROUND

This section provides background information related to the present disclosure which is not necessarily prior art.

Ablation apparatuses and systems are used to perform ablation treatments in which a probe is inserted at or near a target tissue in a patient. The target tissue is typically an abnormal tissue such as a tumor or other growth. The ablation treatment is performed to destroy the target tissue to reduce the likelihood of further growth or spreading of the target tissue in the patient. One type of ablation is thermal ablation. In thermal ablation, the probe that is positioned at or near the target tissue causes the temperature of the target tissue, typically in a localized region proximate the probe, to be elevated to a temperature at which the target tissue is destroyed.

Ablation procedures can be performed in various settings. In some instances, it can be desirable to perform the ablation procedure in a setting in which imaging systems can be used in order to collect images to confirm positioning of the probes in the patient. The images can be used to assist in the guidance and placement of the probes at or near the target tissue(s) and to reduce a likelihood that blood vessels, tissues, organs, or other body structures are damaged during the procedure. Various imaging systems can be used in connection with ablation procedures such as computed tomography (CT) systems, magnetic resonance imaging (MRI) systems, ultrasound systems, x-ray systems, and others. Such imaging systems can be large stationary systems that surround one or more portions of the patient during use. It can be challenging to use and/or position the elements of the ablation apparatus and the imaging systems in close proximity to each other. It can be difficult to move the ablation systems due to their size, shape and/or complexity to a position near the imaging systems and/or other treatment equipment.

In addition, existing ablation systems can be large, cumbersome and difficult to use. The complexity and structure of existing systems may require extensive training and may be highly dependent on the experience level of the operator. Existing ablation systems may also have other drawbacks and designs that may lead to the movement of the ablation probes during treatment. There exists a need, therefore, for improved ablation apparatuses and systems that can be operated in multiple treatment environments for improved treatment outcomes and patient recovery.

### SUMMARY

This section provides a general summary of the disclosure, and is not a comprehensive disclosure of its full scope or all of its features.

In some embodiments of the present disclosure a microwave ablation apparatus is provided. In a first aspect, the present invention provides a microwave ablation apparatus comprises a movable cart comprising a support surface, a portable base unit comprising at least one microwave generator and at least one coolant flow generator, the portable base unit removably positioned on the support surface; and at least one ablation probe assembly configured to couple to the at least one microwave generator and the at least one coolant flow generator.

In one aspect, the movable cart may include an elongated post configured to position the support surface at an elevated position.

In another aspect, the movable cart may include a plurality of wheels mounted to an end of the elongated post opposite to the support surface.

In another aspect, the support surface may include a locator and the portable base unit may include a complimentary locatorthe locator and the complimentary locator configured to position the portable base unit in a desired position on the support surface.

In another aspect, the portable base unit may include a first coolant cartridge port, a first microwave signal port, and a first sensor port.

In another aspect, the portable base unit may include a second coolant cartridge port, a second microwave signal port, and a second sensor port.

In another aspect, the first coolant cartridge port, the first microwave signal port, and the first sensor port are each marked with a first indicator, and the second coolant cartridge port, the second microwave signal port, and the second sensor port are each marked with a second indicator, the first indicator and the second indicator are each configured to identify a different ablation probe assembly.

In another aspect, the first indicator is a first color and the second indicator is a second color.

In another aspect, the portable base unit comprises an adjustable display screen configured to allow user input and the display of one or more operating parameters.

In another aspect, the at least one microwave generator comprises a first microwave generator and a second microwave generator wherein the first microwave generator and the second microwave generator are each operable to independently generate a microwave signal for an independent ablation probe.

In another aspect, the at least one coolant flow generator comprises a pump configured to move coolant through the at least one ablation probe assembly.

In another aspect, the at least one coolant flow generator may include a first coolant flow generator and a second coolant flow generator, the first coolant flow generator and the second coolant flow generator each operable to independently generate flow of a coolant through an independent ablation probe assembly.

In another aspect, the at least one ablation probe assembly comprises an ablation probe, a flexible conduit, a coolant cartridge and a connector.

In another aspect, the connector may be configured to operably connect the at least one ablation probe assembly to the at least one microwave generator and the coolant cartridge is configured to connect the at least one ablation probe assembly to the at least one coolant flow generator.

In an embodiments, the present invention provides, a microwave ablation apparatus comprising a portable base unit comprising a microwave generator port and a coolant port; and an ablation probe assembly comprising a probe, a flexible conduit, a microwave connector and a coolant cartridge, the microwave connector adapted to removably couple the ablation probe assembly to the microwave generator port, and the coolant cartridge adapted to removably couple the ablation probe assembly to the coolant port.

In one aspect, the microwave generator port and the coolant port each include a colored light configured to identify the microwave connector and the coolant cartridge.

In another aspect, the microwave generator port is operably coupled to a microwave generator in the portable base unit and the microwave generator is configured to deliver a microwave signal to the probe.

In another aspect, the coolant port includes a peristaltic pump configured to move coolant through the flexible conduit to the probe.

In an embodiment, the present invention provides a microwave ablation apparatus comprising a portable base unit comprising a housing defining an interior volume; at least one microwave generator positioned in the housing configured to deliver a microwave signal to an ablation probe; and at least one coolant flow generator positioned in the housing configured to move coolant through a conduit to the ablation probe.

In one aspect, the at least one microwave generator comprises a first microwave generator and a second microwave generator, and the at least one coolant flow generator comprises a first coolant flow generator and a second coolant flow generator.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is an isometric view of an example microwave ablation apparatus in accordance with some embodiments of the present disclosure.
FIG. 2 is an illustration of an example microwave ablation apparatus of the present disclosure positioned in a treatment environment near an imaging system.
FIG. 3. is a block diagram of an example base unit in accordance with some embodiments of the present disclosure.
FIG. 4 is an isometric view of the base unit and ablation probe assembly of the example microwave ablation apparatus of FIG. 1.
FIG. 5 is an isometric view of the cart of the example microwave ablation apparatus of FIG. 1.

Corresponding reference numerals indicate corresponding parts throughout the several views of the drawings.

### DETAILED DESCRIPTION

Example embodiments will now be described more fully with reference to the accompanying drawings. For purposes of the description hereinafter, it is to be understood that the embodiments described below may assume alternative variations and embodiments. It is also to be understood that the specific articles, compositions, and/or processes described herein are exemplary and should not be considered as limiting. In the description, relative terms such as "lower," "upper," "horizontal," "vertical,", "above," "below," "up," "down," "top" and "bottom" as well as derivative thereof (e.g., "horizontally," "downwardly," "upwardly," etc.) should be construed to refer to the orientation as then described or as shown in the drawing under discussion. These relative terms are for convenience of description and do not require that the apparatus be constructed or operated in a particular orientation. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

In the present disclosure the singular forms "a," "an," and "the" include the plural reference, and reference to a particular numerical value includes at least that particular value, unless the context clearly indicates otherwise. When values are expressed as approximations, by use of the antecedent "about," it will be understood that the particular value forms another embodiment. As used herein, "about X" (where X is a numerical value) preferably refers to ±10% of the recited value, inclusive. For example, the phrase "about 8" preferably refers to a value of 7.2 to 8.8, inclusive. Where present, all ranges are inclusive and combinable. For example, when a range of "1 to 5" is recited, the recited range should be construed as including ranges "1 to 4", "1 to 3", "1-2", "1-2 & 4-5", "1-3 & 5", "2-5", and the like. In addition, when a list of alternatives is positively provided, such listing can be interpreted to mean that any of the alternatives may be excluded, e.g., by a negative limitation in the claims. For example, when a range of "1 to 5" is recited, the recited range may be construed as including situations whereby any of 1, 2, 3, 4, or 5 are negatively excluded; thus, a recitation of "1 to 5" may be construed as "1 and 3-5, but not 2", or simply "wherein 2 is not included." It is intended that any component, element, attribute, or step that is positively recited herein may be explicitly excluded in the claims, whether such components, elements, attributes, or steps are listed as alternatives or whether they are recited in isolation.

In various embodiments, the present disclosure provides microwave ablation apparatuses and related methods of use. The microwave ablation apparatuses of the present disclosure may include microwave generators that are operable to deliver a microwave signal to an antenna in an ablation probe. The antenna may deliver the microwave energy to a target tissue in a patient in order to raise the temperature of the target tissue to a temperature that destroys the target tissue. The target tissue may be an abnormal tissue such as a tumor or other undesirable growth in a patient. The temperatures of the probe and/or the surrounding tissue may be elevated to temperatures in excess of 55°C and may reach temperatures above 100°C. This thermal energy can be transferred to other elements of the ablation probe assembly such as down the probe away from the distal end, along the cable, to the handle or elsewhere. This transfer of thermal energy can be undesirable because it can cause damage to healthy tissues of the patient and/or can be hazardous to the operators of the ablation equipment.

The microwave ablation apparatuses of the present disclosure include cooling systems to actively cool the ablation probe assemblies. The microwave ablation probe assemblies may include conduits through which a coolant can be moved toward the distal end of the ablation probe. Various suitable coolants may be used such as a saline solution. The saline solution or other coolant may be returned in a coolant loop from the ablation probe to a receptacle for further use and/or circulation through the cable and/or ablation probe. A portion of the saline solution, or other coolant, can be dispensed at the ablation site after it is conveyed via the cooling system to the ablation probe.

The microwave ablation apparatuses of the present disclosure are improvements over existing microwave ablation apparatuses due to the integration of the microwave generators and cooling systems into a portable base unit. The base unit of the microwave ablation apparatus of the present disclosure can be easily moved within a treatment environment and can be placed on a table top, laboratory cart, or other surface. The microwave ablation apparatuses of the present disclosure may also include a portable movable cart on which the base unit can be positioned and moved within the treatment environment. As can be appreciated, the microwave ablation apparatuses of the present disclosure can be easily moved in close proximity to an imaging system in which the patient may be positioned during an ablation treatment. Existing systems are not portable and/or may include multiple different assemblies that can be cumbersome to use and maneuver in a treatment setting.

The microwave ablation apparatuses of the present disclosure are also easier to set-up, use and remove than existing ablation systems. The microwave ablation apparatuses of the present disclosure have simple connectors and ports that allow for connection of the various components without the need for extensive training. The microwave ablation apparatuses are not only easier to use but also reduce the likelihood for errors during set-up and treatment that may otherwise occur with existing systems. The various elements of the microwave ablation apparatuses of the present disclosure can be color-coded or otherwise marked with identifying information to allow a user to intuitively set-up and use the microwave ablation apparatus.

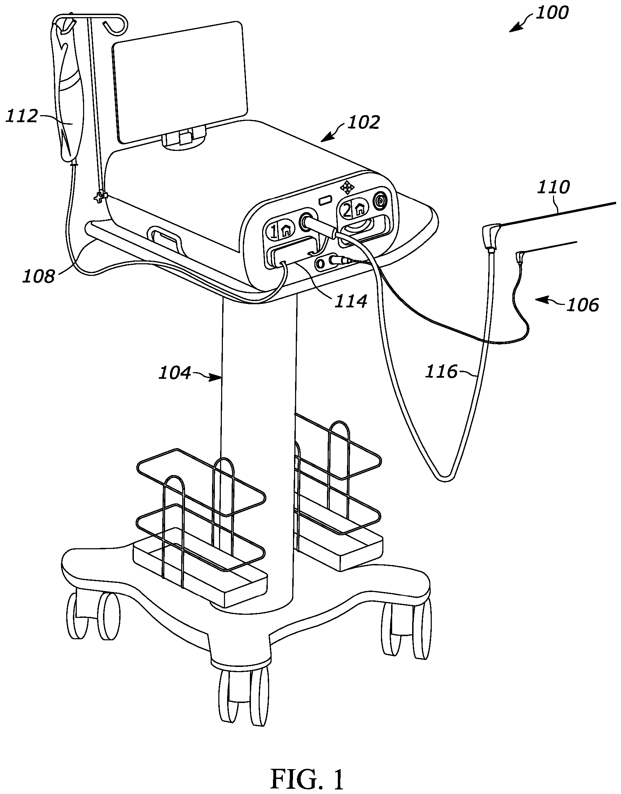

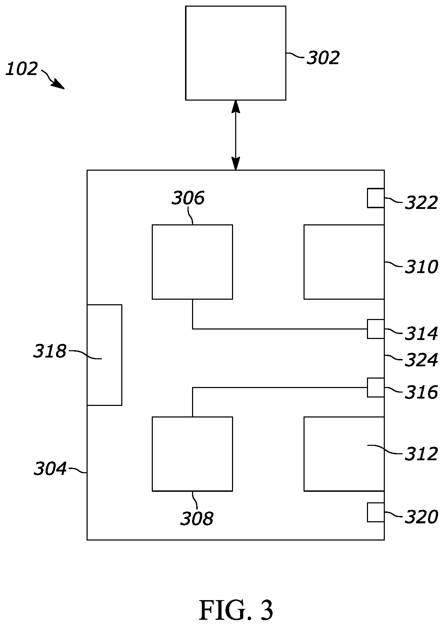

An example microwave ablation apparatus 100 is shown in FIG. 1. The microwave ablation apparatus 100 may include a portable base unit 102, a cart 104, and an ablation probe assembly 106. The base unit 102 can be positioned on the cart 104. As will be further described, the base unit 102 may include a microwave generator and a coolant flow generator (see FIG. 3). In some embodiments, the base unit 102 may have a structure and functionality described in U.S. Patent Application No. TBD, titled CONSOLE ARCHITECTURE FOR MICROWAVE ABLATION UNIT, filed on the same day as the present application to Varian Medical Systems, Inc. The contents of this application are incorporated by reference herein in its entirety. In various embodiments, the ablation apparatus 100 can be operated to perform ablation treatments by sending a microwave signal to an antenna in an ablation probe 110 via cable 116. The antenna may deliver the microwave energy to a target tissue at or near the ablation probe 110. The microwave energy may induce an elevated temperature at the target tissue to destroy the target tissue.

During the ablation treatment, the ablation apparatus 100 may also deliver a coolant from a coolant receptacle 112 to the probe 110 using the coolant flow generator. The coolant cartridge 114 may be inserted into the front face of the base unit 102 and engage a pump to move the coolant from the coolant receptacle 112 to the probe 110 through the cable 116. The movement of the coolant may absorb thermal energy from the cable and/or probe 110 effectively cooling the cable and/or portions of the probe 110.

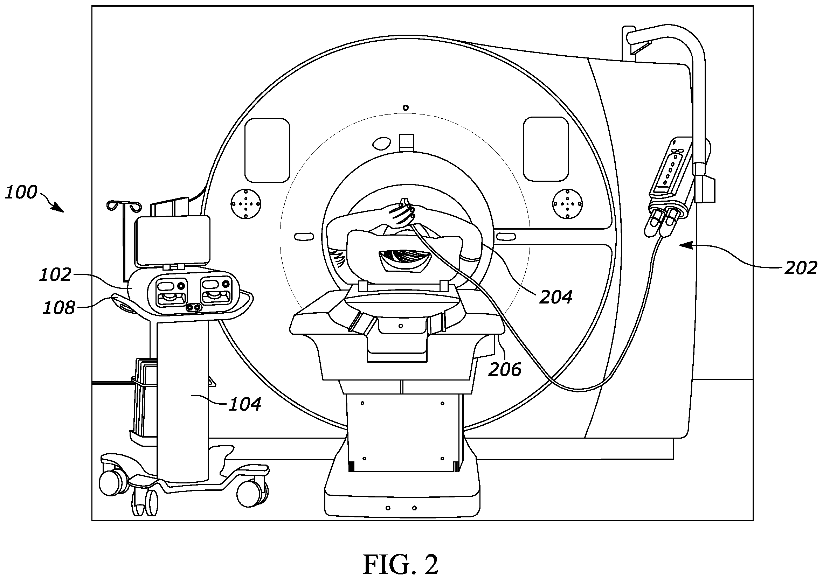

As can be seen in FIG. 2, the base unit 102 and the cart 104 can be easily moved in a hospital, treatment center, or other environment. The cart 104 may include a support surface such as platform 108 that is positioned at an elevated position above the floor to allow access to the functionality of the system. The platform 108 may be positioned, for example, at a height approximately the same as a height of a patient 204. In this example, the patient 204 is positioned on a bed 206 of an imaging system 202. The imaging system 202 may be a CT scanner, MRI machine, X-ray apparatus or other imaging device. With the base unit 102 positioned at a height approximately equal to that of the bed 206 of the imaging system 202, the ablation treatment can be more easily performed. In addition, the base unit 102 can be moved next to the bed 206 so that the length of the cables 116 that may be required to deliver microwave signals and/or coolant from the base unit to the probe 110 can be reduced over ablation systems that are more cumbersome or may need to be located at other positions in the treatment environment.

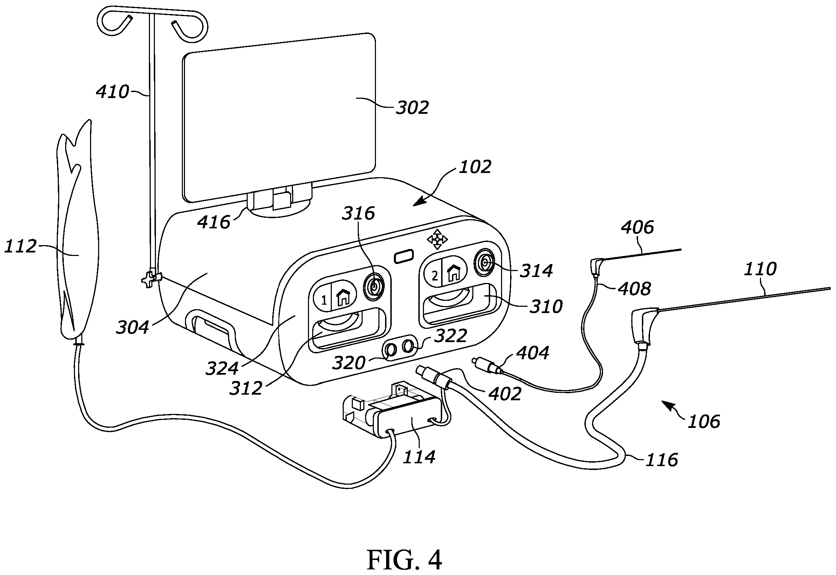

Turning now to FIGs. 3 and 4, the base unit 102 is shown removed from the cart 104. The base unit 102 is portable in that it can be easily separated from the cart 104 without tools and by a single operator. The ablation probe assembly 106 can be removed from the base unit 102 and the display 302 can be folded and laid flat on the top surface of the base unit 102 so that an operator can easily pick up the base unit 102 and move it to a desired location or for storage. The display 302 can be coupled to the base unit 102 via a hinge 416. The hinge 416 can allow the display 302 to be angled, turned, or otherwise adjusted to desired position for viewing during a treatment. The hinge 416 can also allow the display 302 to be laid flat in a position substantially parallel to the top surface of the base unit 102 for storage or during transport.

The base unit 102 may include a housing 304 that is positioned around the inner components of the base unit 102. The housing 304 may include one or more walls that define an interior volume in which the inner components are located. As shown in FIG. 3, the base unit 102 may include a control unit 318, a first microwave generator 306, a second microwave generator 308, a first coolant flow generator 310, a second coolant flow generator 312, a first microwave port 314, a second microwave port 316, a first sensor port 322, and a second sensor port 320. Each of the elements of the base unit 102 may be operably connected to the control unit 318 via suitable wired or wireless connections. The control unit 318 may be a suitable integrated circuit, programmable logic controller (PLC), computing device, or the like that may include a processor and memory to facilitate and/or control the functionality of the base unit 102. The control unit 318 may also be operably connected to the display 302 to allow input from a user and to display one or more operating parameters of the base unit 102.

The first microwave generator 306 and the second microwave generator 308 may be similarly configured to deliver a microwave signal to the ablation probe assembly 106 via the first microwave port 314 and the second microwave port 316, respectively. The first microwave generator 306 and the second microwave generator 308 can include a suitable power supply to provide variable microwave signals having different characteristics so as to heat the target tissue to a predetermined temperature during an ablation treatment. The first microwave generator 306 and the second microwave generator 308 may be independently controllable to allow each generator to generate an independent and separate microwave signal. This may permit an operator to heat respective ablation probes differently or the same as may be desired during an ablation treatment.

The first microwave port 314 and the second microwave port 316 can be similarly configured and can be an electrical connector to allow a mating connector to electrically couple the ablation probe assembly 106 to the respective microwave generator. In some examples, the first microwave port 314 may include a first marking and the second microwave port 316 may include a second marking. The first marking and the second marking may be different to allow a user to differentiate between the ports. The first marking and the second marking may include various suitable identifiers such as a color, a symbol, a letter, a number, a shape, a colored light or others. In some examples, the first marking and the second marking may include multiple identifiers from the previous list of identifiers. The first marking and the second marking can be used so as to guide an operator in the set-up and use of the base unit 102 to ensure that the proper connections are made by the operator. In some embodiments, the ablation probe assembly 106 may have a structure and functionality described in U.S. Patent Application No. TBD, titled MICROWAVE ABLATION PROBE ASSEMBLY AND CABLE STRUCTURE, filed on the same day as the present application to Varian Medical Systems, Inc. The contents of this application are incorporated by reference herein in its entirety.

The first microwave port 314 and the second microwave port 316 may be positioned in a front face 324 of the housing 304. This may allow a user to easily connect an ablation probe assembly 106 to the first microwave port 314 and/or the second microwave port 316. In other examples, the first microwave port 314 and the second microwave port 316 may be positioned at other locations in the housing 304 such as in the side walls, or top surface of the housing 304. While the example base unit 102 shown in FIGs. 3 and 4 include two microwave generators and two microwave ports, it should be appreciated that one generator and port may be included in some examples and more than two generators and ports may be included in other examples.

As further shown in this example, the base unit 102 may include a first sensor port 322 and a second sensor port 320 positioned on the front face 324. This positioning can allow an operator to easily connect one or more sensors to the base unit 102. As shown in FIG. 4, the ablation probe assembly 106 may include a sensor probe 406. The sensor probe 406 may be positioned at or near the target tissue in the patient during an ablation treatment to monitor and/or determine one or more parameters. For example, the sensor probe 406 may include one or more measurement points that can measure an impedance, temperature, moisture, density, or other characteristics of the patient. This measurement information can be transferred via a sensor cable 408 to the base unit 102. The sensor cable 408 may terminate at a sensor connector 404 that can be connected to the base unit 102 at the first sensor port 322 and/or the second sensor port 320. This information can be sent to the control unit 318 to be stored and/or displayed on the display 302. While the example base unit 102 shown in FIG. 4 includes two sensor ports 322, 320, it should be appreciated that the base unit 102 may include less than two sensor ports or more than two sensor ports in other examples. The sensor cable 408 may be provided with or separate from the ablation probe assembly 106 and/or the cable 116. In some examples, the sensors previously described may be incorporated into or positioned on the probe 110. In such examples, the sensors may send signals from the probe 110 to the base unit 102 via the sensor cable 408 coupled to the sensor ports 320, 322. In other examples, the signals and/or information from the sensors may be sent via the cable 116.

The base unit 102 may also include the first coolant flow generator 310 and the second coolant flow generator 312. The first coolant flow generator 310 and the second coolant flow generator 312 may be configured similarly to each other. The first coolant flow generator 310 and the second coolant flow generator 312 may be configured to move coolant through the ablation probe assembly 106 to dissipate or absorb thermal energy and prevent elements of the ablation probe assembly 106 from heating to undesirable levels. The first coolant flow generator 310 and the second coolant flow generator 312 may each include a separate and independent pump that can be operated separately by the control unit 318. Various suitable pumps may be used such as a peristaltic pump.

The first coolant flow generator 310 and the second coolant flow generator 312 may each include a coolant port to allow one or more coolant cartridges 114 to be received in the front face 324 of the base unit 102. In other examples, the coolant ports may be positioned in other regions of the base unit such as in a side wall of the housing 304. The coolant cartridge 114 may include a cradle and tubing that permits the pump to interact with the coolant cartridge 114 to move coolant (e.g., saline) from the coolant receptacle 112 to the cable 116 and/or toward the probe 110. The coolant receptacle 112 can be supported on a hanger 410. The hanger 410 can be an upright hook or bar that suspends the coolant receptacle 112 in a position vertically above the coolant port of the base unit 102. In this example, the hanger 410 is connected to side wall of the housing 304. In other examples, the hanger 410 can be connected to a back wall or top wall of the housing 304. In another example, the hanger 410 can be positioned downward to suspend the coolant receptacle 112 in a position vertically below the base unit 102.

In the example shown, the base unit 102 includes two coolant flow generators. In other examples, the base unit 102 may include one coolant flow generator. In still other examples, the base unit 102 may include more than two coolant flow generators. The example shown in FIG. 4 also shows a single ablation probe assembly 106. It should be appreciated, however, that other ablation systems may include more than one ablation probe assembly 106. The number of ablation probe assemblies may correspond to the number of microwave generators, microwave ports, sensor ports, and/or coolant flow generators. In some embodiments, the coolant flow generators 310, 312 may have a structure and functionality described in U.S. Patent Application No. TBD, titled PUMP AND CASSETTE SYSTEM FOR USE IN MICROWAVE ABLATION, filed on the same day as the present application to Varian Medical Systems, Inc. The contents of this application are incorporated by reference herein in its entirety.

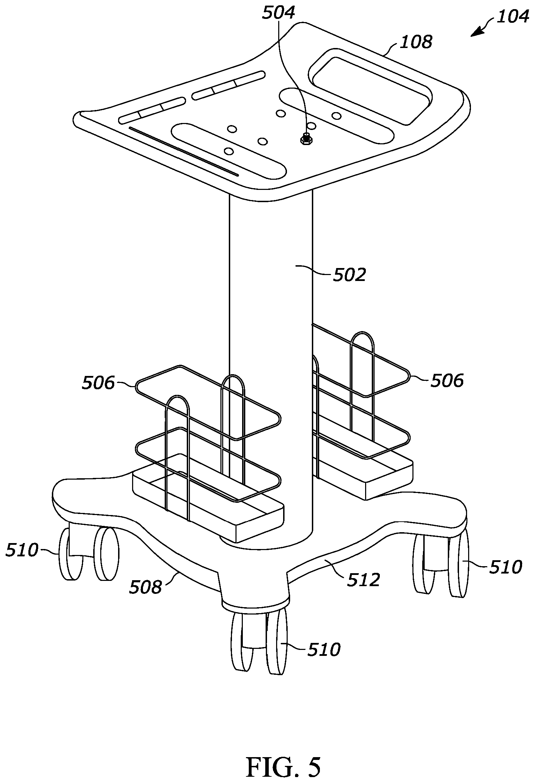

Turning now to FIG. 5, the example cart 104 is shown. The cart 104, in this example, includes the support surface 108, stanchion 502, and base 508. The stanchion 502 is positioned between the support surface 108 and the base 508 to position the support surface 108 at a desired height above the floor. The stanchion 502 can be a suitable post, column or other member to support the base unit 102. In other examples, the stanchion 502 may be adjustable to allow a user to raise or lower the height of the support surface 108 as may be desired.

The base 508 is positioned at or near the floor to support the stanchion in a stable manner. In this example, the base includes a plate 512 and wheels 510. The wheels 510 may be positioned at various locations on the plate 512 to allow the cart 104 to be easily moved in a treatment environment as may be desired. In this example, the base 508 include four wheels 510 with one positioned at each corner of the base 508. In other examples, the base 508 may have other shapes or configurations.

The cart 104 may also include one or more storage bins 506. In this example, two storage bins are positioned on opposite sides of the stanchion 502. The storage bins may configured as wire baskets or may have other shapes or configurations. The storage bins 506 may be used to store records, operating manuals, components, or other items as may be needed during set-up, use or storage of the ablation system 100.

The support surface 108 is positioned at a top of the stanchion 502. The support surface 108 is configured to support and stably hold the base unit 102 in a desired position. The support surface 108 may have a complimentary shape that corresponds to the shape of the base unit 102. In this example, the support surface is curved to compliment the curved shape of the housing 304 of the base unit 102. In other examples, the support surface may be flat or have other shapes. The support surface 108, in this example, has a smooth surface with radiused edges. Such a configuration can allow operators to easily clean and/or sterilize the surfaces of the cart 104 before or after a treatment procedure. Furthermore, the support surface 108 may have openings. The openings may reduce the weight of the cart 104 and also facilitate easy maintenance and cleaning of the support surface 108. The support surface 108 and openings may be configured to create handles that can be grasped or manipulated by a user to facilitate movement and positioning of the cart 104.

As further shown in FIG. 5, the support surface 108 may include a locator 504. In this example, the locator 504 is a cylindrical pin that projects upward away from a top of the support surface 108. The base unit 102 may include an opening that has a complimentary shape to the locator 504. The locator 504 may be received into the opening in the base unit 102. In this manner, the base unit 102 is positioned at a desired location on the support surface 108. In other examples, the locator 504 may be shaped or configured differently than as shown. In other examples, the support surface 108 may be contoured to allow the base unit to be positioned thereon in a desired orientation. In still other examples, the support surface 108 may include more than one locator or have locators having other shapes or sizes. In these or other examples, the locator 504 and complimentary opening in base unit 102 may removably secure, lock, and/or fix the base unit 102 to the support surface 108. In one example, the locator 504 may include a threaded portion that can be secured to a complimentary female threaded portion (e.g., a nut) in the support surface 108. In other examples, the locator 504 may include a clasp, latch, or other feature to removably connect the base unit 102 to the cart 104.

The ablation apparatuses of the present disclosure may be used to perform various treatments and methods. The ablation apparatuses of the present disclosure are improvements over existing systems and methods. The ablation apparatuses of the present disclosure, for example, can be positioned closer to a patient during treatment, particularly when the patient is positioned in an imaging system as previously described.

Furthermore, the ablation apparatuses of the present disclosure are easier to set-up and use than existing ablation systems. In one example, the ablation apparatuses of the present disclosure can be used to set-up and perform an ablation procedure. In such a method, the base unit may be positioned on the cart and moved proximate to an imaging system and patient. The ablation probe may be connected to the base unit by connecting a microwave connector to a microwave port in the base unit. The coolant cartridge may be connected to the coolant flow generator by inserting the coolant cartridge into the coolant port of the base unit. The sensor probe may be connected to the base unit by inserting the sensor connector into the sensor port in the base unit.

The foregoing connections may be performed in response to receiving set-up instructions that may be displayed on the display of the base unit. The markings or indicators on the microwave port, the coolant port, and/or the sensor port may indicate to the operator a sequence of connection and/or a particular corresponding connector. For example, the ports and connectors may have corresponding colors, illuminations, symbols, or the like that indicate to the user the correct location and/or sequence for connection.

The ablation probe and the sensor probe may be positioned at or near a target tissue in the patient. An imaging system may capture images of the target tissue and/or body structures that may be located at or around the target tissue. The operator may use such images to position the ablation probe and/or the sensor probe at a desired position at or near the target tissue.

Once in position, the base unit may operate to deliver a microwave signal to the ablation probe. The coolant flow generator may initiate and deliver a flow of coolant to the ablation probe assembly to dissipate (or absorb) thermal energy from parts of the ablation probe assembly. One or more ablation cycles may be performed to destroy the target tissue. While such ablation is occurring, the sensor probe may collect measurement data characterizing operating parameters of the ablation probe apparatus and/or of the conditions at or near the target tissue. In some instances, temperatures of the ablation probe and the surrounding tissues are monitored. This measurement data may be used to actively control the microwave signal and the flow of coolant to achieve a desired temperature and duration at the target tissue.

While the ablation apparatus 100 may be used to perform the method previously described, other variations of the ablation apparatus 100 may be used as well as other ablation probe apparatuses or systems.

The example methods and apparatuses described herein may be at least partially embodied in the form of computer-implemented processes and apparatus for practicing those processes and/or the described functionality. The disclosed methods may also be at least partially embodied in the form of tangible, non-transient machine readable storage media encoded with computer program code. The media may include, for example, RAMs, ROMs, CD-ROMs, DVD-ROMs, BD-ROMs, hard disk drives, flash memories, or any other non-transient machine-readable storage medium, or any combination of these mediums, wherein, when the computer program code is loaded into and executed by a computer, the computer becomes an apparatus for practicing the method. The methods may also be at least partially embodied in the form of a computer into which computer program code is loaded and/or executed, such that, the computer becomes an apparatus for practicing the methods. When implemented on a general-purpose processor, the computer program code segments configure the processor to create specific logic circuits. The methods may alternatively be at least partially embodied in a digital signal processor formed of application specific integrated circuits for performing the methods.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

The following is a list of illustrative embodiments disclosed herein:

Illustrative embodiment 1: A microwave ablation apparatus comprising: a movable cart comprising a support surface; a portable base unit comprising at least one microwave generator and at least one coolant flow generator, the portable base unit removably positioned on the support surface; and at least one ablation probe assembly configured to couple to the at least one microwave generator and the at least one coolant flow generator.

Illustrative embodiment 2: The microwave ablation apparatus of illustrative embodiment 1, wherein the movable cart comprises an elongated post configured to position the support surface at an elevated position.

Illustrative embodiment 3: The microwave ablation apparatus of illustrative embodiment 2, wherein the movable cart comprises a plurality of wheels mounted to an end of the elongated post opposite to the support surface.

Illustrative embodiment 4: The microwave ablation apparatus of any of the preceding illustrative embodiments, wherein the support surface comprises a locator and the portable base unit comprises a complimentary locator, the locator and the complimentary locator configured to position the portable base unit in a desired position on the support surface.

Illustrative embodiment 5: The microwave ablation apparatus of any of the preceding illustrative embodiments, wherein the portable base unit comprises a first coolant cartridge port, a first microwave signal port, and a first sensor port.

Illustrative embodiment 6: The microwave ablation apparatus of illustrative embodiment 5, wherein the portable base unit comprises a second coolant cartridge port, a second microwave signal port, and a second sensor port.

Illustrative embodiment 7: The microwave ablation apparatus of illustrative embodiment 6, wherein the first coolant cartridge port, the first microwave signal port, and the first sensor port are each marked with a first indicator, and the second coolant cartridge port, the second microwave signal port, and the second sensor port are each marked with a second indicator, the first indicator and the second indicator each configured to identify a different ablation probe assembly.

Illustrative embodiment 8: The microwave ablation apparatus of illustrative embodiment 7, wherein the first indicator is a first color and the second indicator is a second color.

Illustrative embodiment 9: The microwave ablation apparatus of any of the preceding illustrative embodiments, wherein the portable base unit comprises an adjustable display screen configured to allow user input and the display of one or more operating parameters.

Illustrative embodiment 10: The microwave ablation apparatus of any of the preceding illustrative embodiments, wherein the at least one microwave generator comprises a first microwave generator and a second microwave generator, the first microwave generator and the second microwave generator each operable to independently generate a microwave signal for an independent ablation probe.

Illustrative embodiment 11: The microwave ablation apparatus of any of the preceding illustrative embodiments, wherein the at least one coolant flow generator comprises a pump configured to move coolant through the at least one ablation probe assembly.

Illustrative embodiment 12: The microwave ablation apparatus of any of the preceding illustrative embodiments, wherein the at least one coolant flow generator comprises a first coolant flow generator and a second coolant flow generator, the first coolant flow generator and the second coolant flow generator each operable to independently generate flow of a coolant through an independent ablation probe assembly.

Illustrative embodiment 13: The microwave ablation apparatus of any of the preceding illustrative embodiments, wherein the at least one ablation probe assembly comprises an ablation probe, a flexible conduit, a coolant cartridge and a connector.

Illustrative embodiment 14: The microwave ablation apparatus of illustrative embodiment 13, wherein the connector is configured to connect the at least one ablation probe assembly to the at least one microwave generator and the coolant cartridge is configured to connect the at least one ablation probe assembly to the at least one coolant flow generator.

Illustrative embodiment 15: A microwave ablation apparatus comprising: a portable base unit comprising a microwave generator port and a coolant port; and an ablation probe assembly comprising a probe, a flexible conduit, a microwave connector and a coolant cartridge, the microwave connector adapted to removably couple the ablation probe assembly to the microwave generator port, and the coolant cartridge adapted to removably couple the ablation probe assembly to the coolant port.

Illustrative embodiment 16: The microwave ablation apparatus of illustrative embodiment 15, wherein the microwave generator port and the coolant port each include a colored light configured to identify the microwave connector and the coolant cartridge.

Illustrative embodiment 17: The microwave ablation apparatus of any of one of illustrative embodiments 15 or 16, wherein the microwave generator port is coupled to a microwave generator in the portable base unit, the microwave generator configured to deliver a microwave signal to the probe.

Illustrative embodiment 18: The microwave ablation apparatus of any of one of illustrative embodiments 15 through 17, wherein the coolant port includes a peristaltic pump configured to move coolant through the flexible conduit to the probe.

Illustrative embodiment 19: A microwave ablation apparatus comprising: a portable base unit comprising a housing defining an interior volume; at least one microwave generator positioned in the housing configured to deliver a microwave signal to an ablation probe; and at least one coolant flow generator positioned in the housing configured to move coolant through a conduit to the ablation probe.

Illustrative embodiment 20: The microwave ablation apparatus of illustrative embodiment 19, wherein the at least one microwave generator comprises a first microwave generator and a second microwave generator, and the at least one coolant flow generator comprises a first coolant flow generator and a second coolant flow generator.

## Claims

1. A microwave ablation apparatus (100) comprising:
a movable cart (104) comprising a support surface (108);
a portable base unit (102) comprising at least one microwave generator and at least one coolant flow generator, the portable base unit removably positioned on the support surface; and
at least one ablation probe (110) assembly configured to couple to the at least one microwave generator and the at least one coolant flow generator.

2. The microwave ablation apparatus of claim 1, wherein the movable cart comprises an elongated post configured to position the support surface at an elevated position, optionally wherein the movable cart comprises a plurality of wheels mounted to an end of the elongated post opposite to the support surface.

3. The microwave ablation apparatus of claim 1 or claim 2, wherein the support surface comprises a locator and the portable base unit comprises a complimentary locator, the locator and the complimentary locator configured to position the portable base unit in a desired position on the support surface.

4. The microwave ablation apparatus of any preceding claim, wherein the portable base unit comprises a first coolant cartridge port, a first microwave signal port, and a first sensor port.

5. The microwave ablation apparatus of claim 4, wherein the portable base unit comprises a second coolant cartridge port, a second microwave signal port, and a second sensor port, preferably wherein the first coolant cartridge port, the first microwave signal port, and the first sensor port are each marked with a first indicator, and the second coolant cartridge port, the second microwave signal port, and the second sensor port are each marked with a second indicator, the first indicator and the second indicator each configured to identify a different ablation probe assembly, preferably wherein the first indicator is a first color and the second indicator is a second color.

6. The microwave ablation apparatus of any preceding claim, wherein the portable base unit comprises an adjustable display screen configured to allow user input and the display of one or more operating parameters.

7. The microwave ablation apparatus of any preceding claim, wherein the at least one microwave generator comprises a first microwave generator and a second microwave generator, the first microwave generator and the second microwave generator each operable to independently generate a microwave signal for an independent ablation probe, and/orwherein the at least one coolant flow generator comprises a pump configured to move coolant through the at least one ablation probe assembly.

8. The microwave ablation apparatus of any preceding claim, wherein the at least one coolant flow generator comprises a first coolant flow generator and a second coolant flow generator, the first coolant flow generator and the second coolant flow generator each operable to independently generate flow of a coolant through an independent ablation probe assembly.

9. The microwave ablation apparatus of any preceding claim, wherein the at least one ablation probe assembly comprises an ablation probe, a flexible conduit, a coolant cartridge and a connector, preferably wherein the connector is configured to connect the at least one ablation probe assembly to the at least one microwave generator and the coolant cartridge is configured to connect the at least one ablation probe assembly to the at least one coolant flow generator.

10. A microwave ablation apparatus comprising:
a portable base unit comprising a microwave generator port and a coolant port; and
an ablation probe assembly comprising a probe, a flexible conduit, a microwave connector and a coolant cartridge, the microwave connector adapted to removably couple the ablation probe assembly to the microwave generator port, and the coolant cartridge adapted to removably couple the ablation probe assembly to the coolant port.

11. The microwave ablation apparatus of claim 10, wherein the microwave generator port and the coolant port each include a colored light configured to identify the microwave connector and the coolant cartridge.

12. The microwave ablation apparatus of claim 10 or claim 11, wherein the microwave generator port is coupled to a microwave generator in the portable base unit, the microwave generator configured to deliver a microwave signal to the probe.

13. The microwave ablation apparatus of any of claims 10 to 12, wherein the coolant port includes a peristaltic pump configured to move coolant through the flexible conduit to the probe.

14. A microwave ablation apparatus comprising:
a portable base unit comprising a housing defining an interior volume;
at least one microwave generator positioned in the housing configured to deliver a microwave signal to an ablation probe; and
at least one coolant flow generator positioned in the housing configured to move coolant through a conduit to the ablation probe.

15. The microwave ablation apparatus of claim 14, wherein the at least one microwave generator comprises a first microwave generator and a second microwave generator, and the at least one coolant flow generator comprises a first coolant flow generator and a second coolant flow generator.
